# EUROPEAN PATENT SPECIFICATION

(11) **EP 1 661 527 B1**
(45) Date of publication and mention of the grant of the patent: **19.03.2008**
(21) Application number: 04405741.2
(22) Date of filing: 29.11.2004
(51) Int. Cl.: A61B 17/88, A61B 17/92

(54) **Extractor device for an insert for a bone connection element and insert**
Extraktionsvorrichtung für den Einsatz eines Knochenverbindungselementes und Einsatz
Dispositif d'extraction pour un insert d'un élément de connection osseux et un insert

(43) Date of publication of application: 31.05.2006
(73) Proprietor: Stryker Trauma SA, 2545 Selzach (CH)
(72) Inventor: Kugler, Stefan, 3013 Bern (CH); Thomke, Roland, 4512 Bellach (CH); Gasser, André, 4513 Langendorf (CH); Lutz, Christian, 4500 Solothurn (CH)
(74) Representative: Liebetanz, Michael

(56) References cited:
- DE-A1- 19 841 252
- US-A- 6 112 623
- US-A1- 2002 099 386
- DATABASE WPI Section PQ, Week 200367 Derwent Publications Ltd., London, GB; Class P31, AN 2003-704635 XP002330457 & JP 2003 265492 A (REED SHOKAI KK ROBERT) 24 September 2003 (2003-09-24)

## Description

### Technical field of the invention

The invention concerns an extractor device for an insert to be used with a bone connection element usable within an implantable orthopedic device having a load-bearing element such as a bone plate with at least one opening for a fixation element such as a bone screw.

### Technical background of the invention

Such an insert is provided that can be inserted into the opening in a receptacle in which the external shape of the insert is at least partially complementary to the internal shape of the receptacle. The insert has a central through-bore for mounting a body of the fixation element. The implantable orthopedic device has a structure for holding the insert in the receptacle.

A series of implantable orthopedic devices with load-bearing elements, such as bone plates, with openings for the insertion of fixation elements in such load-bearers are known from the prior art. Among them are proposals for the mono-axial as well as poly-axial attachment of fixation elements, particularly screws.

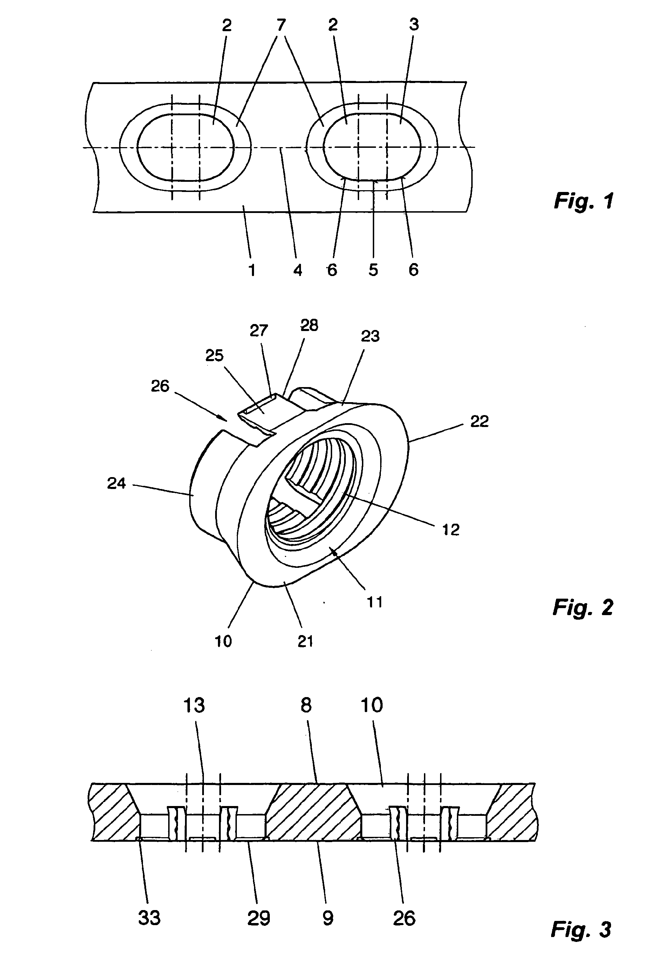

As an example for a device of this type having poly-axial attachment of screws in load-bearing elements is shown in U.S. Patent No. 5,954,722. Other bone plates with inserts are shown in U.S. Patent Nos. 5,976,141 and 5,607,428. One advantageous insert is shown in WO 2004/082493. A removal device adapted to be inserted into a screw via a cannulated screw driver is described in US2002/0099386. Fig. 1 shows a perspective view of an insert to be used with a load-bearing element. FIG. 2 is a sectional side view of a load-bearing element with another insert inserted. The load-bearing elements can be equipped in advance with standard inserts. This enables an easier more cost efficient production of bone plates giving the surgeon the possibility to adapt the bone plate according to his needs. Then it may sometimes be necessary to extract one or the other insert and to replace it by another different insert or to leave the bore empty. The prior art exhibits the disadvantage that there are no extracting tools provided to help the surgeon team to extract inserts.

### Summary of the invention

It is therefore an object of the invention to provide an extractor device allowing to extract easily different inserts from different load-bearing elements.

This objective is fulfilled according to the invention for an extractor device of the aforementioned type with the characteristics of claim 1.

This object is achieved by an extractor device, which can engage the insert in a manner that enables the extraction of said insert from the load-bearing element in a simple and swift movement. Said extraction can be reached for quite a number of different inserts, with different angled positions. Of particular advantage is that the secure extraction of the insert can be assured directly and automatically with the use of the extractor device.

One single extractor device is sufficient to provide the necessary help to extract various inserts with inclined axes as long as the inner bore has a predefined thread. The surgeon can therefore use a bone plate with inserts with predefined angles for the insertion of poly- or monoaxial screws and change one or more of the inserts to orient bone screws at a number of different defined angles in a simple manner.

The following description also shows an insert, which is easy to handle for the surgeon.

Furthermore an insertion device allowing a simple insertion of different inserts into different load-bearing elements is shown.

### Brief description of the drawings

The present invention will be better understood on reading the following detailed description of non-limiting embodiments thereof, and on examining the accompanying drawings, in which:
- FIG. 1: is a top view of a load-bearing element in the form of a bone plate with a row of attachment bores;
- FIG. 2: is a perspective view of a first insert to be used with a load-bearing element;
- FIG. 3: is a sectional side view of a load-bearing element with a second insert inserted;
- FIG. 4: is a perspective view of an extractor device according to one embodiment of the invention;
- FIG. 5: is a side view of the device according to FIG. 4;
- FIG. 6: is a sectional side view of the external sleeve of the extractor device according to Fig. 4;
- FIG. 7: is a perspective view of the inner bar of the extractor device according to Fig. 4;
- FIG. 8: is a sectional side view of the inner bar of FIG. 7;
- FIG. 9: is an enlarged view of the tip of the inner bar of Fig. 7;
- FIG. 10: is a sectional side view of an extractor device according to a second embodiment of the invention in vicinity of an load-bearing element;
- FIG. 11: is a side view of an insertion device according to an embodiment of the invention;
- FIG. 12: is an detail of the tip of the insertion device according to FIG. 11;
- FIG. 13: is a perspective view of a second insert to be used with a load-bearing element;
- FIG. 14: is a sectional side view of the insert according to FIG. 13; and
- FIG. 15: is an enlarged side view showing a detail of the insert according to FIG. 13.

### Detailed description of embodiments of the invention

FIG. 1 shows a top view of a load-bearing element in the form of a plate 1 with a row of attachment bores 2 arranged along the longitudinal direction of plate 1. Fig. 2 shows a perspective view of a first insert 10 to be used with a load-bearing element 1. Bores 2 are through-bores that exhibit an oval central opening 3. At opening 3 there are two sidewalls 5 on opposite sides of axis 4, which extend parallel to the direction of longitudinal axis 4 of plate 1 and extend at right angles to the surfaces of the plate. These parallel sidewalls 5 are connected on both ends by semicircular walls 6, each forming a semi-cylindrical boundary so that together the aforementioned oval opening 3 results.

In other forms of the plate, oval bores 2 can also be provided. Bores 2 can also be elliptical or of a common elongated form. What is essential is the multiplicity of functions for the selection of attachment elements or fasteners made possible by insert 10 shown in FIG. 2. Through the mostly elongated form of plate 1, elongated bores 2 are preferred over circular bores in order to maintain flexibility with the insertion of screws with larger diameters. The bore may also be essentially cylindrical with the disadvantage that the insert 10 has less material for providing inclined holes in the insert 10.

Arranged around the cylindrical, not necessarily circular walls 5, 6 forming opening 3 is chamfered surface area 7, extending and tapering inwardly from the upper surface 8 of plate 1 that faces away from the bone during implantation. The form of this area 7 is preferably part-spherical.

The top surface 8 of load-bearing element 1 is formed somewhat deeper in the side area 18 near bores 2. The same is true for upper edge 15 of sidewall 5, which are shown to be lower (closer to the plate bottom surface) in a direction opposite the bore ends in longitudinal direction 4. Bottom surface 9, which is closest to the bone in insertion during surgery is here locally flat. Normally, plates 1 can exhibit continuous surfaces 8 and undersurfaces 9 which at each point, for the function of positioning on the bone can always be considered to be flat. But here too, positioning on curved or bent surfaces can be provided.

FIG. 2 shows a perspective view of an insert 10 to be used with a load-bearing element 1 according to FIG. 1. Similar characteristics appear in all figures with the same reference numerals. Each insert 10 is designed to be shaped complementary to bore 2 for locking therein with respect to areas 7 and sidewalls 5. Insert 10 has a central bore 11 with an internal threading 12. Internal threading 12 can be cylindrical or slightly conical. Insert 10 can have an area that extends beyond lower surface 9 that is adjacent the bone. In particular, insert 10 has locking mechanisms along its edges. In particular, the locking mechanism can be two projecting rims that engage the underside 9 of plate 1 after the insertion of insert 10. The insert 10, when it is inserted into plate 1, with its extension area, can form a distance spacer with regard to the bone material into which a screw that has been inserted into bore 11 is turned.

It is also possible that at least along the length of the longitudinal axis 4 of the load-bearing element 1 a recess 33 is provided on the bottom surface 9 around opening 2 of the plate 1. This recess can also be provided on the narrow side. In addition, insert 10 is provided with a projecting rim 27 that is arranged in such a way that the bottom of insert 10 does not project beyond lower surface 9. The underside of insert 10 is thus at least flush with the aforementioned surface 9 of the load-bearing element 1.

The reference numeral 21 refers to the surface of insert 10 having a circumference 22 that meets with the edge of area 18 of plate 1. A spherical surface 23 extends downwardly from surface 21 and is shaped so as to have complementary surface contact with surface 7. Semicircular extension 24 extends downwardly from surface 23 and is in conforming contact, without any significant play, with area 6 of bore 2.

The area lying opposite the longitudinal surface 5 consists of a resilient extension 25 on each side, in which, in the preferred embodiment shown, each extension 25 is provided with slots 26. Each extension 25 has, on its lower edge, a projecting rim 27 facing outward from the point of view of the insert with an upward-facing shoulder 28 with an outer edge 29. The surface of rim 27 that is facing toward the bone can be inclined. Extension 25 is also to a certain extent, flexible. In inserting insert 10, extension 25 then slides into bore 2 and is deflected inwardly by the projecting rim 27. Once insert 10 is completely inserted, shoulder 28 slides against underside 9 of plate 1 and locks insert 10 in plate 1. This locking is additionally ensured by the subsequent insertion of a bone screw (not shown).

It is an object of the invention to provide an extractor device being able to extract such a fixed insert 10 from the plate 1. Pressure has to be exerted in such a way that an inward deflection of extension 25 is obtained so that projecting rim 27 is not longer secured under plate 1 or in a recess provided at the bottom of the plate 1. It should be noted that the extractor device has also to be functional if extension 25 and with it projecting rim 27 are not arranged as two extensions 25 on the opposing longer sides of insert 10. The design can also include resilient extensions on the narrow sides of an insert, i.e. corresponding to the location of the semicircular cylindrical extensions 24. Extensions 25 can also be arranged in an alternating manner. There can also be only one or two more extensions.

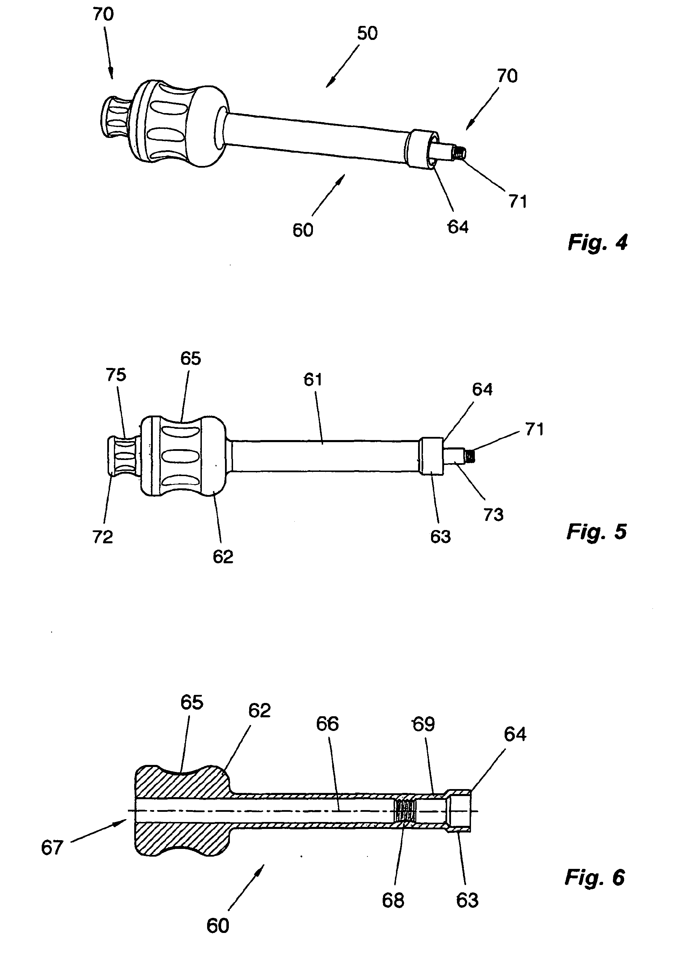

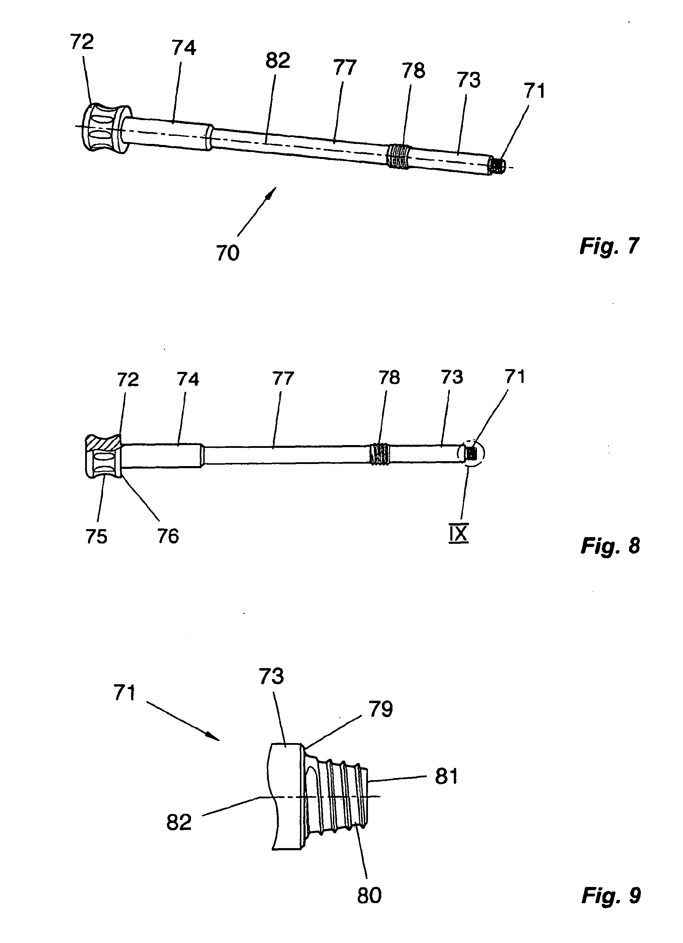

FIG. 4 shows a perspective view of an extractor device 50 according to an embodiment of the invention. Extractor device 50 comprises two different parts, an outer sleeve 60 and an inner bar 70 extending on both sides beyond the through going bore inside the sleeve 60. The outer sleeve 60 is shown in greater detail in Fig. 6, the inner bar 70 is shown in greater detail in Fig. 7 to 9.

FIG. 5 shows a side view of the device 50 according to FIG. 4. The outer sleeve 60 comprises a hollow sleeve part 61 having a first handle part 62 being an integral part of the sleeve 60.

The first handle part 62 comprises a circumferential depression 65 to provide a grip portion for the hand of a user of the device 50. A thickened abutment sleeve 63 is provided on the opposite end of the hollow sleeve part 61 ending in a round abutment ring 64 having a larger diameter than the diameter of the proximal bar section 73 of the inner bar 70. The function of the abutment ring 64 will be explained later on. The length of the external sleeve 60 can be between 150 and 200 Millimeter with a first handle part 62 diameter of 30 to 50 Millimeter and a outer sleeve diameter of e.g. 14 Millimeter.

It can be seen from both, Fig. 4 and 5 that the inner bar 70 extends on both sides of outer sleeve 60. On the side of the first handle part 62 the inner bar 70 comprises a second handle part 72 having a circumferential gripping depression 75. The radius of the second handle part 72 is greater than the inner diameter of the hollow sleeve 60. An engagement thread 71 is provided at the end of the proximal bar section 73 extending over the hollow sleeve 60.

FIG. 6 shows a sectional side view of the external sleeve 60 of the extractor device 50 according to Fig. 4. The hollow sleeve comprises a through-bore with a distal section 66 having a first diameter and extending from the opening 67 near the first handle part 62 towards an internal thread 68. The through-bore comprises on its proximal side a second diameter being smaller than the above-mentioned first inner diameter. The internal thread 68 can have a length of e.g. 10 Millimeter with 5 to 10 full threads. The internal thread 68 is positioned near the proximal end of the sleeve 60, e.g. between 20 and 40 Millimeter from the abutment ring 64. It would also be possible to locate the internal thread 68 near or under the first handle part 62.

Said first diameter, being the inner diameter of a part of the sleeve 60, can be chosen to be 12 Millimeter, said second diameter of the distal end portion 69 can be chosen to be 11 Millimeter. The enlarged section 63 can have a diameter of 14 Millimeter.

FIG. 7 shows a perspective view of the inner bar 70 of the extractor device 50 according to Fig. 4. FIG. 8 shows a sectional side view of the inner bar 70 of FIG. 7. The inner bar 70 comprises a first distal bar portion 74 having an outer diameter similar to the first inner diameter of the hollow sleeve 60 so that the bar 70 can be inserted with little play into the hollow sleeve 60 from the side of the opening 67. The distal bar portion 74 ends in an abutment flange 76 of the gripping depression 75. On the opposite end of the distal bar portion 74 a middle bar portion 77 has a thinner cylindrical portion. The middle bar portion 77 is followed by a threaded portion 78 having a longitudinal length which is preferably identical to the inner threaded portion 68 of the outer sleeve 60. The location of said thread also depends on the location of the inner threaded portion 68. If said portion 68 is located near or under the first handle part 62 then the threaded portion 78 of the inner bar 70 is located near or in the area of the distal bar portion 74.

In the embodiment shown in the drawings, the threaded portion 78 is proximally followed by the proximal inner bar section 73. Said inner bar section 73 is followed by a tip portion 71 of the inner bar 70, which is shown in FIG. 9 in an enlarged view.

The tip portion 71 comprises four full threads of a thread 80 ending in a flat surface 81. The thread 80 is conical and is connected with the inner bar section 73 via a chamfered flange 79. The direction of the threading 80 is identical to the diretion of the thread 78. The main axis 82 of the bar 70 coincides with the axis 82 of the thread 80 and with the longitudinal main axis of the sleeve 60. The advantage of a conical thread 80 is the easier fixation of the bar onto inserts 10 with damaged threads 12.

The function of the device 50 is as follows. The device is assembled through insertion of the bar 70 into the hollow sleeve 60 through the distal opening 67 within the sleeve 60. The thread 78 of the bar 70 engages the inner thread 68 of the hollow sleeve 60 and is rotated until the tip 71 is extending over the end of the hollow shaft 60 or until the threads 68 and 78 come free one from the other. Both threads 68 and 79 are right-handed, i.e. the thread 78 advances when turned clockwise with respect to its mating part 68. The flange 76 of the second handle part 72 can ultimately abut against the first handle part 62 of the hollow sleeve 60. The thread 78 may then no longer be in engagement with thread 68.

The bar 70 is then screwed into the insert 10 by turning the handle 72, i.e. the conical thread 80 is engaging the internal threading 12 of the insert 10. Preferably the abutment flange 79 of the tip is coming into contact with the upper surface 21 of the insert 10. Then the abutment ring 64 is advanced to come into contact with the surface 8 of the plate 1 beside the insert 10, i.e. the abutment ring 64 does not contact surface 21 but touches the plate 1 outside the circumference 21 of the insert 10. This contact is possible independent from the angle and direction of the insert bore 11 as discussed above. The relative advancement of sleeve 60 in relation to the bar 70 can be a longitudinal movement or comprising a rotation when the threads 68 and 78 come into engagement. For a rotational movement the handle part 62 is rotated against the handle part 72 of the inner bar, which is maintained in its position. The internal thread 12 of the insert 10 is also right-handed.

When the abutment ring 64 comes in contact with the upper surface 8 of the plate 1 (and not with the insert 10), then the extractor sleeve 60 is further rotated in the direction opposite to the above mentioned rotation of the bar 70. Said rotation retracts the rod 73 further into the sleeve 60 and therefore exerts a force in the longitudinal direction 82 of the extractor device 50 on the insert 10 against the plate 1. This leads to the situation that engagement means 25 of the insert 10 are overcome and the insert 10 will be extracted. The advantage of right-handed threads 68 and 78 resides in the fact that the rotation to extract the insert 10 additionally blocks the thread 12 of the insert 10 in thread 80 of the tip 71.

In another embodiment (not shown) the threads 68 and 78 may be left-handed. Then the surgeon has the usual anti-clockwise movement to extract the insert; however, it is then possible that the thread 12 of the insert 10 becomes no longer fastened to the conical thread 80 of the inner bar 70.

It is clear that the abutment ring 64 can be of a different form than having a circular shape. It may have a polygonal abutment surface or the plane of the abutment surface can comprise an angle with the plane perpendicular to the longitudinal direction 82 of the bar 70.

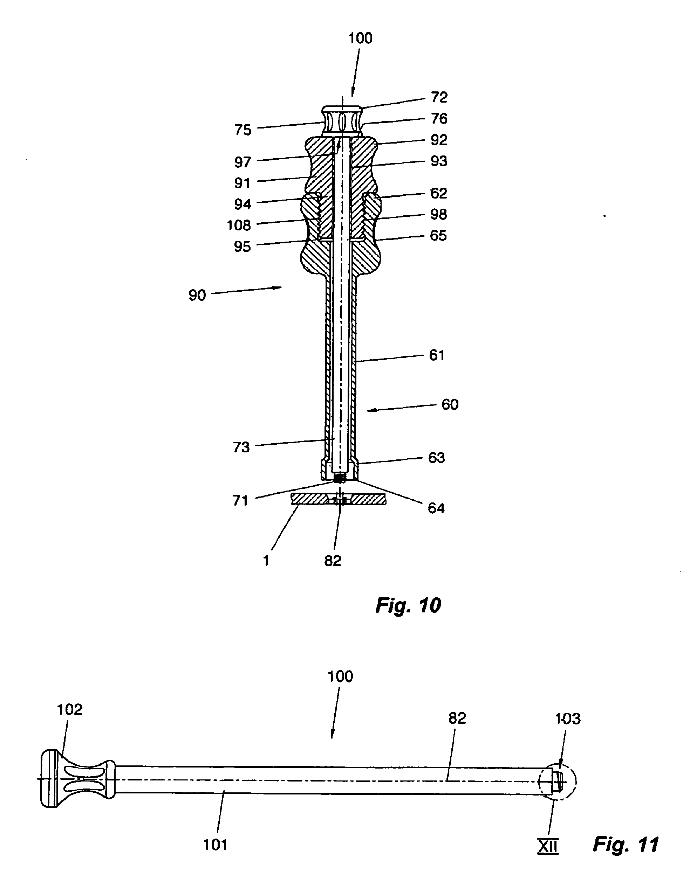

FIG. 10 shows a sectional side view of an extractor device 90 according to a second embodiment of the invention in vicinity of an load-bearing element 1. Extractor device 90 comprises three different parts, an outer sleeve 60 and an inner bar 70 extending on both sides beyond the through going bore inside the sleeve 60. Additionally there is an intermediate element 91 essential to the function of the device.

The outer sleeve 60 comprises a hollow sleeve part 61 having a first handle part 62 being an integral part of the sleeve 60. The first handle part 62 comprises a circumferential depression 65 to provide a grip portion for the hand of a user of the device 50. A thickened abutment sleeve 63 is provided on the opposite end of the hollow sleeve part 61 ending in a round abutment ring 64 having a larger diameter than the diameter of the proximal bar section 73 of the inner bar 70. The function of the abutment ring 64 is identical as explained above.

The inner bar 70 extends on both sides of outer sleeve 60. On the side of the first handle part 62 the inner bar 70 comprises a second handle part 72 having a circumferential gripping depression 75. Between the two handle parts 62 and 72 the intermediate element 91 is introduced.

The intermediate element 91 has a through bore 93 for the inner bar 70. The intermediate element 91 comprises a grip portion 92 being able to be in abutment against the handle part 72 of inner bar 70 on one side and with the handle part 62 of the sleeve 60 on the other side. On the side of the handle part 72 the intermediate element 91 comprises an opening 97 in the flat upper surface to receive the inner bar 70. On the side of the handle part 62 the intermediate element 91 comprises a smaller central projection 94 with an external thread 98.

Sleeve 60 incorporates in the handle part 62 a recess 95 to accommodate the projection 94, wherein the recess 95 comprises an internal thread 108.

As in the first embodiment of an extractor device 50, an engagement thread 71 is provided at the end of the proximal bar section 73 extending over the hollow sleeve 60.

Inner bar 70 of the extractor device 90 comprises a bar portion 74 having an outer diameter similar to the inner diameter of the hollow sleeve 60 and similar to the inner diameter of the intermediate element 91 so that the bar 70 can be inserted with little play into the intermediate element 91 and into the hollow sleeve 60 from the side of the opening 67 / 97. The bar portion 74 ends in an abutment flange 76 of the gripping depression 75.

The tip portion 71 of extractor device 90 can be the same as the tip portion 71 of extractor device 50.

The function of the device 90 is as follows. The device is assembled through insertion of the intermediate element 91 into the hollow sleeve 60 through the distal opening 67 within the sleeve 60. The thread 98 of the intermediate element 91 engages the inner thread 108 of the hollow sleeve 60 and is rotated until the handle part 92 abuts against the handle part 62 or the projection 94 reaches the depth of the recess 95. Both threads 98 and 108 are right-handed. The bar 70 is then entered into intermediate element 91 and hollow sleeve 60 and screwed into the insert 10 by turning the handle 72, i.e. the conical thread 80 is engaging the internal threading 12 of the insert 10. Then the abutment ring 64 is advanced to come into contact with the surface 8 of the plate 1 beside the insert 10, i.e. the abutment ring 64 does not contact surface 21 but touches the plate 1 outside the circumference 21 of the insert 10.

The handle part 92 of the intermediate element 91 is rotated against the handle part 62 of the hollow sleeve 60, which is maintained in its position. The intermediate element 91 and the hollow sleeve 60 are moving apart, while the inner bar 70 just follows, because the handle part 72 is in abutment with the intermediate element 91. Said rotation of intermediate element 91 indirectly retracts the rod 73 further into the sleeve 60 and therefore exerts a force in the longitudinal direction 82 of the extractor device 90 on the insert 10 against the plate 1. This leads to the situation that engagement means 25 of the insert 10 are overcome and the insert 10 will be extracted. In the case of the second embodiment it is advantageous to use right-handed threads 80 and 12 whereas threads 98 and 108 are left-handed. Then the rotation to extract the insert 10 blocks the insert 10 in thread 80 of tip 71.

However in all embodiments it is possible to switch the handedness of any pair of threads.

Upon extraction of an insert 10, said insert 10 nearly disappears within the hollow thickened abutment sleeve 63.

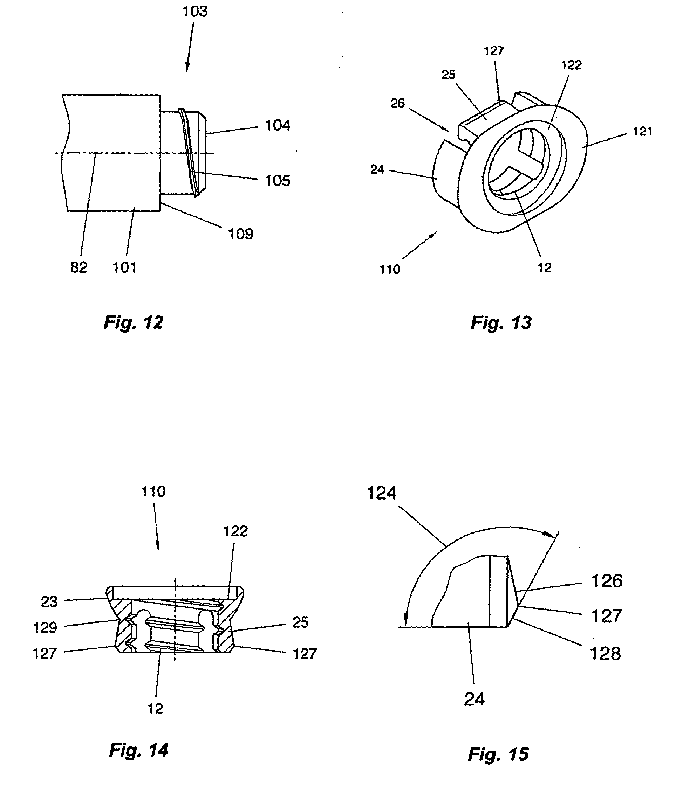

FIG. 11 shows a side view of an insertion device 100 according to an embodiment of the invention and FIG. 12 is an detail of the tip of the insertion device 100 according to FIG. 11. Insertion device 100 is very similar to inner bar 70, having a bar 101 with a handle part 102 at one end, whereas the other free end of the bar 101 comprises a cylindrical tip 103 with a smaller diameter than the bar 102. Said tip 103 comprises one single thread 105 and ends with a flat bottom surface 104, which can be chamfered for easier insertion. The tip 103 is positioned within the opening of an insert 10 and the thread 105 engages the complementary thread 12 of the insert 10. It is clear that due to the shortness of the tip 103 the resilient extensions of insert 10 are not in the vicinity of the tip 103 but spaced apart in the longitudinal direction 82 of the insertion tool 100. It is possible that the distance between the thread 12 and the upper surface 21 of the insert 10 is such that said surface 21 can abut against the abutment flange 109 of the bar 101.

Initially the insert 10, 20 to be inserted into a plate 1 is affixed to the insertion device 100 as mentioned above. Then the device 100 with the insert 10, 20 at its tip is pushed against and into the plate 1 until the locking extensions 25 of the insert 10, 20 are pushed beyond the smallest waist part in the hole 2 of a plate 1. Then the insertion device 100 can be rotated in the opposite direction to free the insert 10, 20 from its tip. This can be done directly, because the inserts 10, 20 are oblong.

FIG. 13 shows a perspective view of a second insert 110 to be used with a load-bearing element 1. FIG. 14 is a sectional side view of the insert 110 according to FIG. 13 and FIG. 15 is an enlarged side view showing a detail of the resilient extension 25 of the insert according to FIG. 13. Insert 110 comprises a flat upper surface 121 at its circumference wherein on the inside of surface 121 a step-like recess 122 is provided. The resilient extension 25 on each side is separated as within the embodiment according to Fig. 1 from the semicircular extensions by two slots 26. The resilient extension 25 comprises a special triangular form when seen from the side or in a cross section.

On the inside the resilient extension 25 comprises the inner thread 12 which can be oriented in different ways and angles. On the outside the insert 110 has a waist 129, i.e. an area with minimum exterior diameter. Towards the upper side 121 of the insert 110 there is the larger spherical surface 23 or another complementary surface for the bore 2 in plate 1. Towards the bottom side there is provided, on the resilient extensions 25, a thickened region 127. In the embodiment shown the thickened region 127 has a thickest part near the lower end of the extension 25, i.e. the cross section through the extension 25 always shows a triangle form. Advantageously the form of the plate 1 is complementary, i.e. the plate has at least on its sides a recess to accommodate the thickened region 127. The chamfered lower end 128 facilitates the introduction of the insert 110 into a plate 1. The angle of the surface 128 in relation to the bottom surface is 120 degree (reference numeral 124). The triangle form 127 also facilitates the extraction of the insert 110 because the inclined surface 126, e.g. with an angle of 30 degree to the vertical axis of the insert 110, can slide on the complementary inclined surface of the plate 1.

Although the described drawings already show a whole series of possible configurations of the invention, the invention is and should be limited only by the parameters of the attached claims.

The advantage of the invention is that it offers the surgeon using a plate 1 with conventional standard bores 2 and inserted inserts 10, 110 the possibility of adapting the plurality of angularly-stable mono-axial bores by replacement of inserts, and furthermore, that this is made possible intra-operatively.

### Reference Numerals

- 1: plate
- 2: bores
- 3: central opening
- 4: axis
- 5: parallel side walls
- 6: semicircular walls
- 7: surface area
- 8: upper surface
- 9: bottom surface
- 10: insert
- 11: central bore
- 12: internal threading
- 15: upper edge
- 18: side area
- 21: surface
- 22: circumference
- 23: spherical surface
- 24: semicircular extension
- 25: resilient extension
- 26: slot
- 27: projecting rim
- 28: upward facing shoulder
- 29: outer edge
- 33: recess
- 50: extractor device
- 60: outer sleeve
- 61: hollow sleeve part
- 62: first handle part
- 63: abutment sleeve
- 64: abutment ring
- 65: circumferential depression
- 66: distal section
- 67: opening
- 68: internal thread
- 69: distal end portion
- 70: inner bar
- 71: engagement thread
- 72: second handle part
- 73: proximal bar section
- 74: distal bar portion
- 75: circumferential depression
- 76: abutment flange
- 77: middle bar portion
- 78: threaded portion
- 79: chamfered flange
- 80: thread
- 81: flat surface
- 82: main axis
- 90: extractor device
- 91: intermediate element
- 92: grip portion
- 93: through bore
- 94: central projection
- 95: recess
- 97: opening
- 98: external thread
- 100: insertion device
- 101: bar
- 102: handle part
- 103: cylindrical tip
- 104: bottom surface
- 105: thread
- 108: internal thread
- 109: abutment flange
- 110: insert
- 121: flat upper surface
- 122: step-like recess
- 124: angle
- 126: inclined surface
- 127: thickened region
- 128: surface
- 129: waist

## Claims

1. An extractor device (50, 90) for an insert (10, 20) to be used with a bone connection element usable within an implantable orthopedic device having a load-bearing element (1) with at least one opening (2) for a fixation element with said insert (10, 20), the extractor device (50, 90) comprising:
- an outer hollow sleeve (60), the hollow sleeve comprising a distal introduction opening (67) and a proximal abutment surface (64) being able to engage a surface portion (8) of the load-bearing element (1) around the insert (10), the hollow sleeve (60) further comprising an inner thread (68, 108) between said distal introduction opening (67) and said proximal abutment surface (64),
- an inner bar (70), the bar having a diameter enabling the insertion of the bar (70) into said distal introduction opening (67) of the hollow sleeve (60) and comprising a proximal thread (71) at its proximal end to engage the thread (12) of the insert (10) to be extracted,
- wherein, alternatively, the inner bar (70) further comprises an outer thread (78) or an intermediate element (91) is provided with a central bore to accommodate said inner bar (70) and comprising an outer thread (98),
- wherein the outer thread (78, 98) is arranged to engage said inner thread (68, 108) of the hollow sleeve (60),
- said outer thread (78, 98) being positioned on the bar (70) or the intermediate element (91) in a longitudinal distance from the proximal thread (71) to enable the proximal thread (71) to extend beyond the proximal abutment surface (64) to be threaded in the insert (10).

2. The device as set forth in claim 1, wherein the proximal thread (71) of the bar (70) is a conical thread.

3. The device as set forth in claim 1 or 2, wherein an abutment flange (79) connects the thread (71) with a larger bar section (73) of the inner bar (70).

4. The device as set forth in claims 1, 2 or 3, wherein the inner bar (70) comprises at its distal end a handle part (72) having an abutment flange (76).

5. The device as set forth in one of claims 1 through 4, wherein the hollow sleeve (60) comprises at its proximal end an enlarged abutment section (63) to engage a surface (2) of the load-bearing element (1).

6. The device as set forth in one of claims 1 through 5, wherein the hollow sleeve (60) comprises at its distal end a hollow handle part (62).

7. The device as set forth in one of claims 1 through 6, wherein the threads (68 and 78) of sleeve (60) and bar (70), respectively, are located in the proximal first third of the device (50).

8. The device as set forth in one of claims 1 through 7, wherein the threads (68 and 78; 108 and 98) of sleeve (60) and bar (70) or intermediate element (91) have the same handedness than the thread (80) of the tip (71) of the inner bar (70).

9. The device as set forth in one of claims 1 through 8, wherein the abutment surface (64) is a cylindrical ring, an elliptical ring or a polygonal strip.

## Patentansprüche

1. Extraktionsvorrichtung (50, 90) für einen Einsatz (10, 20) für den Einsatz mit einem Knochenverbindungs-Element, einsetzbar innerhalb einer implantierbaren orthopädischen Einrichtung mit einem Last tragenden Element (1) mit mindestens einer Öffnung (2) für ein Befestigungs-Element mit dem besagten Einsatz (10, 20), wobei die Extraktionsvorrichtung (50, 90) umfasst:
- eine äussere hohle Hülse (60), wobei die hohle Hülse eine distale Einführungsöffnung (67) und eine proximale Anschlagfläche (64) aufweist, die fähig ist, in einen Oberflächenabschnitt (8) des Last tragenden Elementes (1) um den Einsatz (10) herum einzugreifen, wobei die hohle Hülse (60) weiterhin ein Innengewinde (68, 108) zwischen der besagten distalen Einführungsöffnung (67) und der besagten proximalen Anschlagsfläche (64) umfasst;
- einen inneren Stab (70), wobei der Stab einen Durchmesser hat, der die Einführung des Stabes (70) in die besagte distale Einführungsöffnung (67) der hohlen Hülse (60) gestattet und ein proximales Gewinde (71) an seinem proximalen Ende umfasst, um in das Gewinde (12) des auszuziehenden Einsatzes (10) einzugreifen;
- wobei in alternativer Weise der innere Stab (70) weiterhin ein Aussengewinde (78) oder ein Zwischenelement (91) aufweist, welches mit einer zentralen Bohrung versehen ist, um den besagten Innenstab (70) aufzunehmen, und ein äusseres Gewinde (98) aufzuweisen;
- wobei das Aussengewinde (78, 98) so ausgestaltet ist, um das besagte Innengewinde (68, 108) der hohlen Hülse (60) einzugreifen;
- wobei das besagte Aussengewinde (78, 98) so auf dem Stab (70) oder dem Zwischenelement (91) in einem longitudinalen Abstand von dem proximalen Gewinde (71) positioniert ist, um es dem proximalen Gewinde (71) zu gestatten, sich über die proximale Anschlagfläche (64) zu erstrecken, um in den Einsatz (10) eingeschraubt zu werden.

2. Vorrichtung nach Anspruch 1, wobei das proximale Gewinde (71) des Stabes (70) ein konisches Gewinde ist.

3. Vorrichtung nach Anspruch 1 oder 2, wobei ein Anschlagflansch (79) das Gewinde (71) mit einem grösseren Stababschnitt (73) des Innenstabes (70) verbindet.

4. Vorrichtung nach einem der Ansprüche 1, 2 oder 3, wobei der Innenstab (70) an seinem distalen Ende einen Handgriffabschnitt (72) mit einem Anschlagflansch (76) aufweist.

5. Vorrichtung nach einem der Ansprüche 1-4, wobei die hohle Hülse (60) an ihrem proximalen Ende einen vergrösserten Anschlagabschnitt (63) aufweist, um in eine Oberfläche (2) des Last tragenden Elementes (1) einzugreifen.

6. Vorrichtung nach einem der Ansprüche 1-5, wobei die hohle Hülse (60) an ihrem distalen Ende über einen hohlen Handgriffabschnitt (62) verfügt.

7. Vorrichtung nach einem der Ansprüche 1-6, wobei die Gewinde (68, 78) der Hülse (60) und des Stabes (70) jeweils in dem proximalen ersten Drittel der Vorrichtung (50) angeordnet sind.

8. Vorrichtung nach einem der Ansprüche 1-7, wobei die Gewinde (68 und 78; 108 und 98) der Hülse (60) und des Stabes (70) oder des Zwischenelementes (71) dieselbe Händigkeit haben, wie das Gewinde (80) der Spitze (71) des Innenstabes (70).

9. Vorrichtung nach einem der Ansprüche 1-8, wobei die Anschlagoberfläche (64) ein zylindrischer Ring, ein elliptischer Ring oder ein polygonaler Streifen ist.

## Revendications

1. Dispositif d'extraction (50, 90) pour un insert (10, 20) pour l'utilisation avec un élément de connection osseux qui peut être utilisé avec un dispositif orthopédique implantable avec un élément (1) supportant des charges avec au moins une ouverture (2) pour un élément de fixation avec ledit insert (10, 20), où le dispositif d'extraction (50, 90) comprend:
- une douille (60) extérieure creuse, où la douille creuse comprend une ouverture (67) d'introduction distale et une surface butoir (64) proximale, qui est capable d'engager une section de surface (8) de l'élément supportant des charges autour de l'insert (10), où la douille (60) creuse comprend en outre un filetage (78, 108) interne entre ladite ouverture (67) d'introduction distale et ladite surface butoir (64) proximale;
- une barre (70) interne, où la barre comporte un diamètre permettant l'insertion de la barre (70) dans l'ouverture d'introduction (67) distale de la douille (60) creuse et comprend un filetage (71) proximal à son bout proximal pour engager le filetage (12) de l'insert (10) qui est à extraire;
- où soit la barre (70) interne comprend en outre un filetage (78) extérieur, soit un élément intermédiaire (91) est prévu avec un perçage central pour accommoder ladite barre (70) interne et comprend un filetage (98) extérieur;
- où le filetage (78, 98) extérieur est arrangé pour engager ledit filetage (68, 108) intérieur de la douille (60) creuse;
- où ledit filetage (78, 98) extérieur étant positionné sur la barre (70) ou sur l'élément (91) intermédiaire dans une distance longitudinale du filetage (71) proximal pour permettre au filetage (71) proximal de s'étendre au delà de la surface butoir (64) proximale pour être vissé dans l'insert (10).

2. Dispositif selon la revendication 1, où le filetage (71) proximal de la barre (70) est un filetage conique.

3. Dispositif selon l'une des revendications 1 ou 2, où une bride (79) butoir connecte le filetage (71) avec une section de barre plus large (73) de la barre (70) interne.

4. Dispositif selon l'une quelconque des revendications 1, 2 ou 3, où la barre (70) interne comprend à son bout distal une section de poignée (72) comportant une bride butoir (76).

5. Dispositif selon l'une quelconque des revendications 1 à 4, où la douille (60) creuse comprend à son bout proximal une section butoir (63) élargie pour engager une surface (2) de l'élément (1) supportant des charges.

6. Dispositif selon l'une quelconque des revendications 1 à 5, où la douille (60) creuse comprend à son bout distal une section de poignée (62) creuse.

7. Dispositif selon l'une quelconque des revendications 1 à 6, où les filetages (68, 78) de la douille (60) et de la barre (70), respectivement, sont localisés dans le premier tiers proximal du dispositif (50).

8. Dispositif selon l'une quelconque des revendications 1 à 7, où les filetages (68 et 78; 108 et 98) de la douille (60) et de la barre (70) ou de l'élément intermédiaire (71) possèdent la même chiralité que le filetage (80) de la pointe (71) de la barre (70) interne.

9. Dispositif selon l'une quelconque des revendications 1 à 8, où la surface butoir (64) est un anneau cylindrique, un anneau elliptique ou une bande polygonale.
